(19) Europäisches Patentamt
European Patent Office
Office européen des brevets

(11) Veröffentlichungsnummer: **0 132 730**
**B1**

(12) # EUROPÄISCHE PATENTSCHRIFT

(45) Veröffentlichungstag der Patentschrift: **18.05.88**

(51) Int. Cl.⁴: **C 07 D 249/08**, A 01 N 43/653

(21) Anmeldenummer: **84108291.0**

(22) Anmeldetag: **14.07.84**

(54) **3-(1,2,4-Triazol-1-yl)-1-propene.**

(30) Priorität: **27.07.83 DE 3327036**

(43) Veröffentlichungstag der Anmeldung: **13.02.85 Patentblatt 85/7**

(45) Bekanntmachung des Hinweises auf die Patenterteilung: **18.05.88 Patentblatt 88/20**

(84) Benannte Vertragsstaaten: **AT BE CH DE FR GB IT LI NL SE**

(56) Entgegenhaltungen:
**EP-A-0 052 424**
**EP-A-0 056 125**
**EP-A-0 057 365**
**EP-A-0 063 099**
**EP-A-0 094 167**
**DE-A-3 130 215**
**DE-A-3 218 130**
**US-A-4 315 017**

**CHEMICAL ABSTRACTS, Vol. 97, No. 7, 16. August 1982, Columbus, Ohio, USA. H. Ehrhardt, H. Mildenberger: "Addition of CH-acidic compounds to the carbon-carbon triple bond", S. 644, Spalte 2, Abstract No. 55 738e**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Weissmüller, Joachim, Dr., Sillerstrasse 49, D-5600 Wuppertal 11 (DE)**
Erfinder: **Krämer, Wolfgang, Dr., Am Eckbusch 39/45, D-5600 Wuppertal 1 (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen 1 (DE)**
Erfinder: **Reinecke, Paul, Dr., Lessingstrasse 11, D-5090 Leverkusen 3 (DE)**

**EP 0 132 730 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue 3-(1,2,4-Triazol-1-yl)-1-propene, mehrere Verfahren zu ihrer Herstellung sowie ihre Verwendung als Fungizide.

Es ist bereits bekannt geworden, daß neben manchen Azolylethylverbindungen, wie beispielsweise dem 1-(2,6-Dichlorbenzyloxy)-1-(4-chlorphenyl)-2-(1,2,4-triazol-1-yl)-ethan (vgl. DE-A-2 547 953 auch bestimmte 3-Azolyl-1-propene, wie beispielsweise das cis- oder das trans-1,2-Bis-(2,4-dichlorphenyl)-3-(1,2,4-triazol-1-yl)-1-propen (vergl. DE-A-2 652 313) fungizide Eigenschaften besitzen. Siehe auch UJ-A-4 315 017.

Die Wirkung dieser Azolderivate ist jedoch in bestimmten Indikationsbereichen insbesondere bei niedrigen Aufwandmengen und - konzentrationen nicht immer völlig zufriedenstellend.

In de EP-A-0 094 167 werden 3-(1,2,3-Triazol-1-yl)-1-propene und deren Verwendung als fungizide beschrieben. Ind der DE-A-3 218 130 werden 3-(1,2,4-Triazol-1-yl)-1-propene und deren Verwendung als Zwischenprodukte zur Herstellung von Wirkstoffen mit fungiziden Eigenschaften beschrieben.

Es werden aber keine Verbindungen dieses Typs offenbart, in denen das Kohlenstoffatom in α-Position zum Triazolyl-Rest eine oder zwei Methylgruppen trägt. Ferner werden keine Stoffe erwähnt, in denen in Nachbarstellung zum Triazolyl-Rest eine $CH_2$-Gruppe steht und gleichzeitig an den Kohlenstoffatomen der Doppelbindung andere Reste als Alkyl mit 1 bis 4 Kohlenstoffatomen oder gegebenenfalls substituiertes Phenyl stehen (vgl. DE-A-3 218 130, Seite 13 unten und Seite 14 oben).

Es wurden neue 3-(1,2,4-Triazol-1-yl)-1-propene der allgemeinen Formel (I),

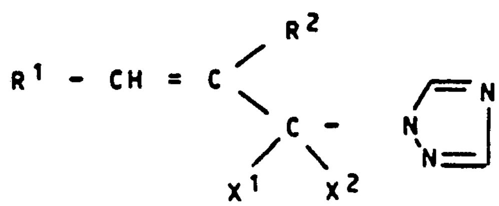

(I)

in welcher

a) entweder

$X^1$ und $X^2$ für Methyl stehen,

$R^1$ für Methyl, Ethyl n- und i-Propyl n-, i-, s- und t-Butyl, n-Pentyl, neo-Pentyl, n- und i-Hexyl-, für Allyl, Butenyl, für jeweils gegebenenfalls einfach oder mehrfach durch Methyl substituierts Cyclopentyl, Cyclohexyl, Cyclohexenyl oder Cycloheptyl steht, oder für jeweils gegebenenfalls ein- bis dreifach, gleich oder verschieden subsituiertes Phenyl oder Benzyl steht, wobei als Substituenten zu nennen sind:

Hydroxy, Flour, Chlor, Brom, Cyano, Nitro, Methyl, Methoxy, Methylthio, Methoxycarbonyl, Trifluormethyl, Triflourmethoxy, Trifluormethylthio, Ethyl, Ethoxy, Ethylthio, Ethoxycarbonal, n- und i-Propyl, Isopropyloxy, n-, i-, s- und t-Butyl, Cyclohexyl, Dimethylamino, Diethylamino, Acetyloxy, Acetamido, N-Methylacetamido sowie durch Flour, Chlor oder Methyl, ein- bis dreifach, gleich oder verschieden substituiertes Phenyl oder Phenoxy, und

$R^2$ für Methyl, Ethyl, n- und i-Propyl, n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$-C- Rest, für jeweils

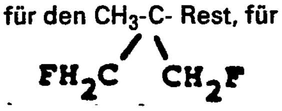

gegebenenfalls ein- bis dreifach, gleich oder verschieden substituiertes Phenyl oder Benzyl oder für einen im Phynylkern ein- bis dreifach, gleich oder verschieden substituierten

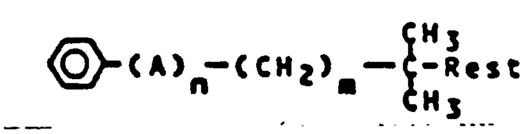

steht, wobei als Phenylsubstituenten jeweils die bei $R^1$ genannten infrage kommen; wobei

A für Sauerstoff oder Schwefel steht,

m für eine Zahl 0, 1 oder 2 steht, und

n für eine Zahl von 0 oder 1 steht,

jedoch mit der Maßgabe, daß $R^1$ und $R^2$ nicht gleichzeitig für gegebenenfalls substituiertes Phenyl stehen dürfen,

oder

b) $X^1$ für Wasserstoff steht,

$X^2$ für Methyl steht,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cycloheptyl steht, oder für jeweils gegebenenfalls ein- bis dreifach, gleich oder verschieden substituiertes Phenyl oder Benzyl steht, wobei als Substituenten zu nennen sind:

Hydroxy, Flour, Chlor, Brom, Cyano, Nitro, Methyl, Methoxy, Methylthio, Methoxycarbonyl, Trifluormethyl, Triflourmethoxy, Trifluormethylthio, Ethyl, Ethoxy, Ethylthio, Ethoxycarbonal, n- und i-Propyl, Isopropyloxy, n-, i-, s- und t-Butyl, Cyclohexyl, Dimethylamino, Diethylamino, Acetyloxy, Acetamido, N-Methylacetamido sowie durch Flour, Chlor oder Methyl, ein- bis dreifach, gleich oder verschieden substituiertes Phenyl oder Phenoxy, und

$R^2$ für Methyl, Ethyl, n- und i-Propyl, n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$-C- Rest, für jeweils

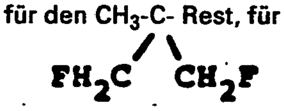

gegebenenfalls ein- bis dreifach, gleich oder verschieden substituiertes Phenyl oder Benzyl oder für einen im Phynylkern ein- bis dreifach, gleich oder verschieden substituierten

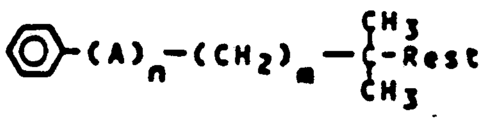

steht wobei als Phenylsubstituenten jeweils die bei $R^1$ genannten infrage kommen; wobei

A für Sauerstoff oder Schwefel steht,

m für eine Zahl 0, 1 oder 2 steht, und

n für eine Zahl von 0 oder 1 steht,

jedoch mit der Maßgabe, daß $R^1$ und $R^2$ nicht gleichzeitig für gegebenenfalls substituiertes Phenyl stehen dürfen,

oder

c) $X^1$ und $X^2$ für Wasserstoff steht,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cycloheptyl steht, oder für jeweils gegebenenfalls ein- bis dreifach, gleich oder verschieden substituiertes Phenyl oder Benzyl steht, wobei als Substituenten zu nennen sind:

Hydroxy, Flour, Chlor, Brom, Cyano, Nitro, Methyl, Methoxy, Methylthio, Methoxycarbonyl, Trifluormethyl, Triflourmethoxy, Trifluormethylthio, Ethyl, Ethoxy, Ethylthio, Ethoxycarbonal, n- und i-Propyl, Isopropyloxy, n-, i-, s- und t-Butyl, Cyclohexyl, Dimethylamino, Diethylamino, Acetyloxy, Acetamido, N-Methylacetamido sowie durch Flour, Chlor oder Methyl, ein- bis dreifach, gleich oder verschieden substituiertes Phenyl oder Phenoxy, und

$R^2$ für n- und i-Pentyl n- und i-Hexyl, n- und i-Heptyl, Allyl, Propenyl, Butenyl, für jeweils gegebenenfalls einfach oder mehrfach durch Methyl substituiertes Cyclopentyl, Cyclohexyl, Cyclohexenyl oder Cycloheptenyl steht oder für den Rest

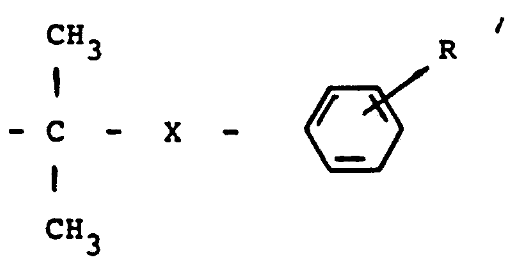

steht, wobei

R' für Wasserstoff, Hydroxy, Fluor, Chlor, Brom, Cyano, Nitro, Methyl, Methoxy, Methylthio, Trifluormethyl, Trifluormethoxy, Trifluormethylthio, Ethyl, Ethoxy, Ethylthio, n- und i-Propyl, Isopropyloxy, n-, i-, s- und t-Butyl oder Cyclohexyl steht und

X für $CH_2$ Sauerstoff, Schwefel steht,

sowie deren pflanzenverträgliche Säureadditionssalze und Metallsalzkomplexe gefunden.

Die Verbindungen der Formel (I) können als geometrische Isomere oder Isomerengmische unterschiedlicher Zusammensetzung anfallen. Sowohl die reinen Isomeren als auch die Isomerengemische werden erfindungsgemäß beansprucht.

Weiterhin wurde gefunden, daß man die neuen 3-(1,2,4-Triazol-1-yl)-1-propene der allgemein Formel (I), sowie deren pflanzenverträgliche Säureadditionssalze und Metallsalzkomplexe erhält, wenn man

a) 3-Halogen- bzw. 3-Sulfonyloxy-1-propene der allgemeinen Formel (II),

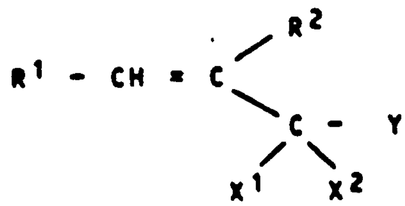

(II)

in welcher
$R^1$, $R^2$, $X^1$ und $X^2$ die oben angegebene Bedeutung haben
und
Y für Chlor, Brom Methansulfonyloxy oder p-Toluolsulfonyloxy steht,
mit 1,2,4-Triazol gegebenenfalls in Gegenwart eines Verdünnungsmittels und gegebenenfalls in Gegenwart eines Säurebindemittels umsetzt; oder daß man
b) 1,2,4- triazol-1-ylmethylketone der Formel

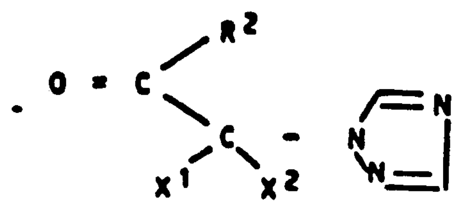

(III)

in welcher
$R^2$, $X^1$ und $X^2$ die oben angegebene Bedeutung haben, mit Phosphonsäureestern bzw. Phosphinoxiden der Formel

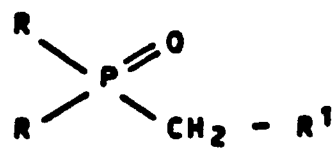

(IVa)

in welcher
$R^1$ die oben angegebene Bedeutung hat und
R für Methoxy, Ethoxy oder Phenyl steht,
oder alternativ mit Triphenylalkylidenphosphoranen der Formel

$(C_6H_5)_3P = CH - R^1$ (IVb)

in welcher
$R^1$ die oben angegebene Bedeutung hat,
in Gegewart einer Verdünnungsmittels und in Gegenwart einer Base im Sinne einer "Witting-Olefinierung" umsetzt, und gegebenenfalls noch an die so erhaltenen Verbindungen der Formel (I) eine Säure oder ein Metallsalz addiert.

Schließlich wurde gefunden, daß die neuen 3-(1,2,4-Triazol-1-yl)-1-propene der Formel (I) sowie deren pflanzenverträgliche Säureadditions-Salze und Metallsalz-Komplexe sehr gute fungizide Eigenschaften besitzen.

Überraschenderweise zeigen die erfindungsgemäßen Wirkstoffe der Formel (I) eine bessere fungizide Wirksamkeit als die aus dem Stand der Technik bekannten Triazolverbindungen 1-(2,6-Dichlorbenzyloxy)-1-(4-chlorphenyl)-2-(1,2,4-triazol-1-yl)-ethan oder cis- bzw. trans-1,2-Bis-(2,4-dichlorphenyl)-3-(1,2,4-triazol-1-yl)-propen, welches chemisch und wirkungsmäßig naheliegende Verbindungen sind. Die erfindungsgemäßen Stoffe stellen somit eine wertvolle Bereicherung der Technik dar.

Im einzelnen seien außer den bei den Herstellungsbeispielen genannten Verbindungen, die folgenden Verbindungen der allgemeinen Formel (I) genannt:

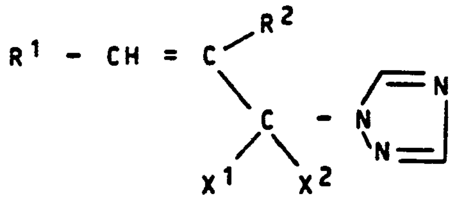
(I)

| $R^1$ | $R^2$ | $X^1$ | $X^2$ |
|---|---|---|---|
| $C_2H_5$ | 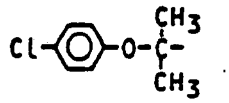 | H | H |
| n-$C_3H_7$ | 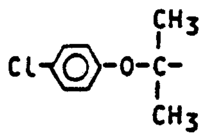 | H | H |
| i-$C_3H_7$ | 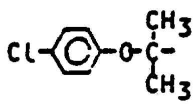 | H | H |
| n-$C_3H_7$ | 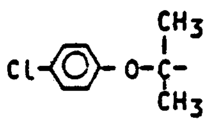 | H | H |
| 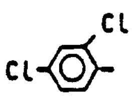 | 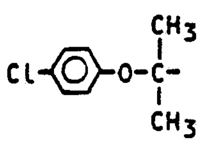 | H | H |
| $C_2H_5$ | 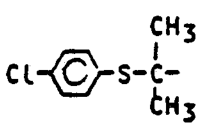 | H | H |
| n-$C_3H_5$ | 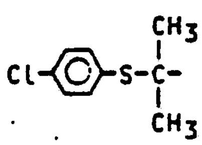 | H | H |
| i-$C_3H_7$ | 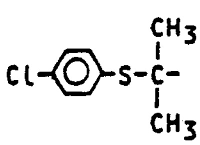 | H | H |
| 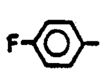 | 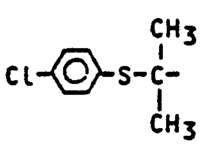 | H | H |
| n-$C_3H_7$ | 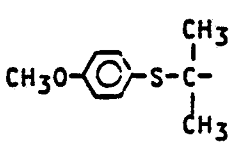 | H | H |
| 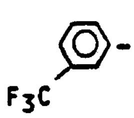 | 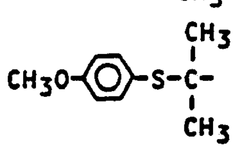 | H | H |

| | | | |
|---|---|---|---|
| 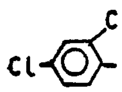 | n-$C_7H_{15}$ | H | H |
| $C_2H_5$ | 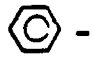 | $CH_3$ | $CH_3$ |
| n-$C_3H_7$ | 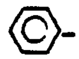 | $CH_3$ | $CH_3$ |
| 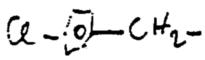 | 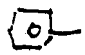 | $CH_3$ | $CH_3$ |
| 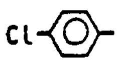 | 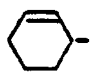 | H | H |
| 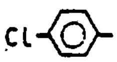 | $H_3C$-CH=CH- | H | H |
| n-$C_5H_{11}$ | 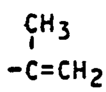 | H | H |
| n-$C_3H_7$ | 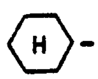 | H | H |
| 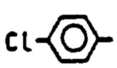 | 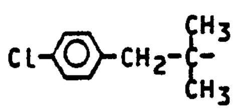 | H | H |
| 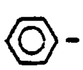 | 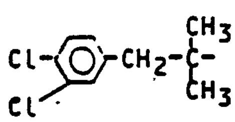 | H | H |
| 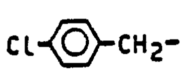 | $CH_3$ | $CH_3$ | $CH_3$ |

Verwendet man beispielsweise 2-Brommethyl-1-(2,4-dichlorphenyl)-3,3-dimethyl-1-buten und 1,2,4-Triazol als Ausgangsstoffe, so läßt sich der Reaktionsablauf des erfindungsgemäßen Verfahrens a) durch das folgende Formelschema darstellen:

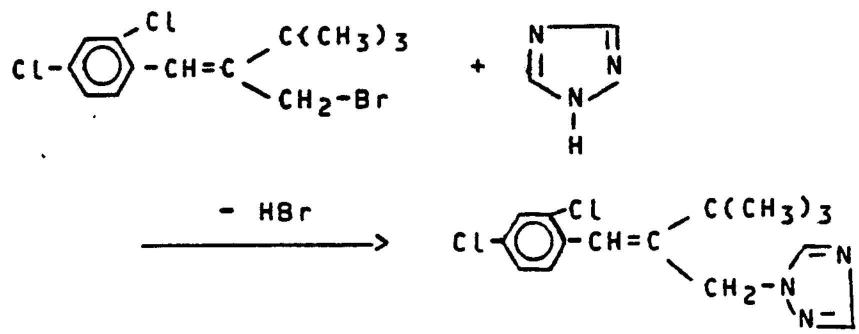


Die zur Durchführung des erfindungsgemäßen Verfahrens a) als Ausgangsstoffe benötigten 3-Halogen- bzw. 3-Sulfonyloxy-1-propene sind durch die Formel (II) allgemein definiert. In dieser formel (II) stehen $X^1$, $X^2$, $R^1$ und $R^2$ für diejenigen Reste, die schon bei der Beschreibung der entsprechenden Reste der erfindungsgemäßen Wirkstoffe der Formel (I) genannt wurden. Y steht für Chlor, Brom, Methansulfonyloxy oder p-Toluolsulfonyloxy.

Die 3-Halogen- bzw. 3-Sulfonyloxy-1-propene der Formel (II) sind teilweise bekannt [vergl. z. B. Chemisches Zentralblatt 1927, II, 1811; Compt.Rend.Sci. 232, 1762 (1951)].

Man erhält sie, wenn man Allylalkoholderivate der Formel (V),

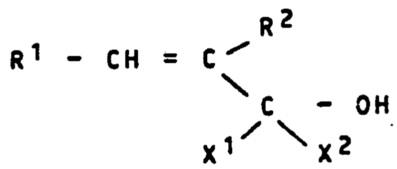
(V)

in welcher

$R^1$, $R^2$, $X^1$ und $X^2$ die oben angegebene Bedeutung haben,

entweder mit Halogenierungsmitteln wie beispielsweise Phosphorpentachlorid, Phosphortribromid oder Thionylchlorid in bekannter weise gegebenenfalls in Gegenwart eines Verdünnungsmittels wie beispielsweise Dichlormethan, Chloroform oder Tetrachlorkohlenstoff bei Temperaturen zwischen 0°C und 80°C umsetzt zu den Halogenderivaten der Formel (IIa),

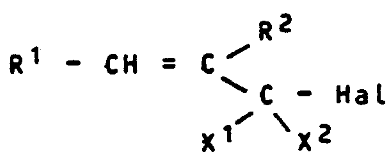
(IIa)

$R^1$, $R^2$, $X^1$ und $X^2$ die oben angegebene Bedeutung haben und

Hal für Chlor oder Brom steht,

oder mit Sulfonsäurechloriden der Formel (VI)

$R' - SO_2 - Hal'$ (VI)

in welcher

R' fü Methyl oder p-Tolyl steht und

Hal' für Chlor oder Brom steht,

ebenfalls in bekannter Weise gegebenenfalls in Gegenwart eines Verdünnungsmittels wie beispielsweise Dichlormethan und gegebenenfalls in Gegenwart eines Säurebindemittels, wie beispielsweise Pyridin bei Temperaturen zwischen 20°C und 100°C umsetzt zu den Sulfonyloxyderivaten der Formel (IIb),

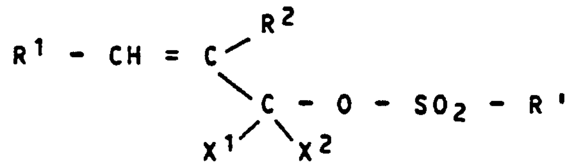

(IIb)

in welcher
$R^1$, $R^2$, $X^1$, $X^2$ und R' die oben angegebene Bedeutung haben.

Alternativ erhält man Verbindungen der Formel (IIa),

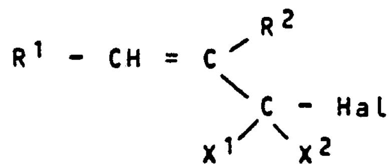

(IIa)

in welcher
$R^1$, $R^2$, $X^1$, $X^2$ und Hal die oben angegebene Bedeutung haben, wenn man Ketone der Formel (VII)

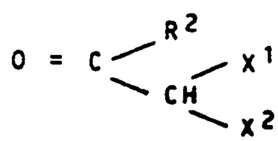

(VII)

in welcher
$R^2$, $X^1$ und $X^2$ die oben angegebene Bedeutung haben,
zunächst in einer 1. Stufe mit Phosphonsäureestern bzw. Phosphinoxiden der allgemeinen Formel (IVa),

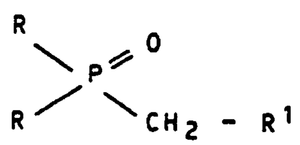

(IVa)

in welcher
$R^1$ die oben angegebene Bedeutung hat und
R für Methoxy, Ethoxy oder Phenyl steht,
in Gegenwart einer Base, wie beispielsweise Kalium-t-butylat und in Gegenwart eines Verdünnungsmittels, wie beispielsweise Toluol bei Temperaturen zwischen 50°C und 150°C im Sinne einer "Wittig-Olefinierung" umsetzt zu den Olefinen der Formel (VIII)

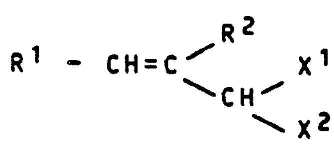

(VIII)

in welcher
$R^1$, $R^2$, $X^1$ und $X^2$ die oben angegebene Bedeutung haben,
und diese in einer 2. Stufe in bekannter Weise z. B. mit Chlor oder mit N-Bromsuccinimid, gegebenenfalls in Gegenwart eines Verdünnungsmittels, wie beispielsweise 1,1,2,2-Tetrachlorethan oder Tetrachlorkohlenstoff und gegebenenfalls in Gegenwart eines Katalysators wie beispielsweise Azo-diisobutyrodinitril bei Temperaturen zwischen 70°C und 250°C halogeniert.

Allylalkoholderivate der Formel (V),

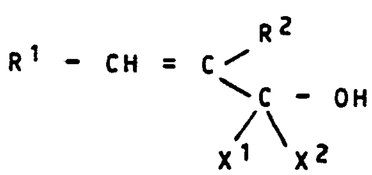

(V)

in welcher
$R^1$, $R^2$, $X^1$ und $X^2$ die oben angegebene Bedeutung haben,

erhält man, wenn man α,β-ungesättigte Carbonsäureester oder Aldehyde der Formel (IX),

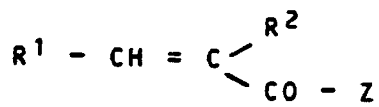 (IX)

in welcher

$R^1$ und $R^2$ die oben angegebene Bedeutung haben und

Z für Wasserstoff, Methoxy oder Ethoxy steht,

in üblicher weise entweder mit Lithiumaluminiumhydrid oder mit Natriumborhydrid, gegebenenfalls in Gegenwart eines Katalysators, wie beispielsweise Lithiumiodid, gegebenenfalls in Gegenwart eines Verdünnungsmittels wie beispielsweise Tetrahydrofuran bei Temperaturen zwischen -20° C und + 50° C reduziert zu den Allylalkoholderivaten der Formel (Va),

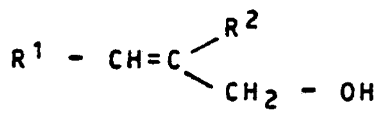 (Va)

in welcher

$R^1$ und $R^2$ die oben angegebene Bedeutung haben,

oder ebenfalls in üblicher Weise mit metallorganischen Methylverbindungen, wie beispielsweise Methyllithium oder Methylmagnesiumbromid gegebenenfalls in Gegenwart eines Verdünnungsmittels wie beispielsweise Ether bei Temperaturen zwischen -80° C und +50° C umsetzt zu den Allylalkoholderivaten der Formel (Vb),

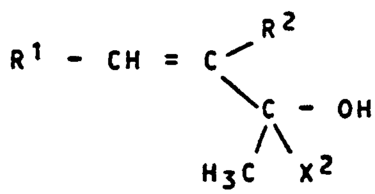 (Vb)

in welcher

$R^1$, $R^2$ und $X^2$ die oben angegebene Bedeutung haben.

Die α,β-ungesättigten Carbonsäureester oder Aldehyde der Formel (IX) sind bekannt, oder können nach bekannten Verfahren in analoger Weise erhalten werden (vergleiche z. B. Can. J. Chem. 49, 2143 [1971]; J. Amer. Chem. Soc. 80, 4949 [1958]; J. Chem. Soc. 1961, 3160; Liebigs. Ann. Chem. 658, 21 [1962]; Helv. chim. Acta 34, 1482 [1951]; J. Org. Chemistry 22, 33 [1957]; J. Amer. Chem. Soc. 69, 2605 [1947]; J. org. Chem. 16, 867 [1951]; J. Amer. Chem. Soc. 67, 1432 [1945]; J. org. Chemistry 23, 803 [1958]; J. Amer. Chem. Soc. 92, 226 und 6635 [1970]; J. org. Chemistry 26, 4278 [1961]; J. Chem. Soc. 1969, 2799).

Die Phosphorverbindungen der Formel (IVa) und (IVb), und die Sulfonylhalogenide der Formel (VIII) sind allgemein bekannte Verbindungen der organischen Chemie.

Die zur Durchführung des erfindungsgemäßen Verfahrens b) als Ausgangsstoffe benötigten 1,2,4-Triazol-1-yl-methylketone sind durch die Formel (III) allgemein definiert. In dieser Formel (III) stehen $R^2$, $X^1$ und $X^2$ für diejenigen Reste, die schon bei der Beschreibung der entsprechenden Reste der erfindungsgemäßen Wirkstoffe der Formel (I) genannt wurden.

Die 1,2,4-Triazol-1-ylmethylketone der Formel (III) sind bekannt (vergl. z. B. DE-A-2 951 164; DE-A-2 431 407; DE-A-3 048 266; DE-A-3 104 311; DE-A-3 219 041; DE-A-3 222 221; DE-A-3 232 737.

Als Verdünnungsmittel kommen für das erfindungsgemäße Verfahren a) inerte organische Lösungsmittel infrage. Hierzu gehören vorzugsweise chlorierte Kohlenwasserstoffe, wie z. B. Dichlormethan, Chloroform, Tetrachlorkohlenstoff oder Chlorbenzol, Amide, wie Dimethylformamid, Dimethylacetamid oder N-Methylformanilid, Nitrile, wie Acetonitril oder Propionitril, sowie die hochpolaren Lösungsmittel Dimethylsulfoxid, Sulfolan oder Hexamethylphosphorsäuretriamid.

Das erfindungsgemäße Verfahren a) kann in Gegenwart eines Säurebindemittels vorgenommen werden. Man kann alle üblicherweise verwendbaren anorganischen oder organischen Säurebinder zugeben, wie Alkalicarbonate, z. B. Natriumcarbonat und Kaliumcarbonat, oder niedere tertiäre Alkylamine, Cycloalkylamine oder Aralkylamine, z. B. Triethylamin, N,N-Dimethylcyclohexylamin und N,N-Dimethylbenzylamin. Ebenfalls möglich ist ein entsprechender Überschuß an 1,2,4-Triazol.

Die Reaktionstemperaturen können bei der erfindungsgemäßen Verfahren a) in einem größeren Bereich variiert werden. Im allgemeinen arbeitet man zwischen 0° C und 150° C, vorzugsweise zwischen 35° C und 90° C.

Zur Durchführung des erfindungsgemäßen Verfahrens a) setzt man auf 1 Mol der Verbindungen der

allgemeinen Formel (II) vorzugsweise 1 bis 30 Mol 1,2,4-Triazol ein. Die Isolierung der Verbindungen der allgemeinen Formel (I) erfolgt nach üblichen Methoden.

Als Verdünnungsmittel für das erfindungsgemäße Verfahren b) kommen ebenfalls inerte organische Lösungsmittel infrage. Hierzu gehören vorzugsweise aliphatische oder aromatische Kohlenwasserstoffe, wie beispielsweise n- oder i-Butan, Pentan, Hexan, Cyclohexan, Benzol, Toluol oder Xylol, Ether, wie beispielsweise Diethylether, Dimethoxyethan, Tetrahydrofuran, Dioxan oder Diethylenglykoldimethylether, Amide, wie beispielsweise Dimethylformamid, 2-Pyrrolidon oder Hexamethylphosphorsäuretriamid sowie Sulfoxide, wie beispielsweise Dimethylsulfoxid.

Das erfindungsgemäße Verfahren b) wird üblicherweise in Gegenwart einer Base durchgeführt. Bevorzugt sind starke organische oder anorganische Basen, beispielsweise Hydride, wie Lithiumhydrid, Natriumhydrid, Calziumhydrid, Amide, wie Natriumamid oder Lithiumdiisopropylamid, Alkoholate, wie Natriummethylat, Natriumethylat, Kaliumethylat, Kaliumtbutylat oder auch metallorganische Verbindungen wie Nethyllithium, Propyllithium, Butyllithium, Phenyllithium, Triphenylmethylnatrium oder Mesitylmagnesiumbromid.

Die Reaktionstemperaturen können bei dem erfindungsgemäßen Verfahren b) in einem größeren Bereich variiert werden. Im allgemeinen arbeitet man zwischen -20°C und + 150°C, vorzugsweise zwischen 0°C und 120°C.

Zur Durchführung des erfindungsgemäßen Verfahrens b) setzt man pro Mool 1,2,4-Triazol-1-ylmethylketon der Formel (III) im allgemeinen 1,0 bis 1,3 Mol, vorzugsweise äquimolare Menge an Phosphonsäureester bzw. Phosphinoxid der Formel (IVa) oder 1,0 bis 1,3 Mol, vorzugsweise äquimolare Menge an Triphenylalkylidenphosphoran der Formel (IVb) und im allgemeinen 1,0 bis 2,0 Mol, vorzugsweise 1,0 bis 1,3 Mol an Base ein.

Üblicherweise legt man den Phophonsäureester bzw. das Phophinoxid der Formel (IVa) oder das Triphenylalkylidenphosphoran der Formel (IVb) zusammen mit der Base in dem entsprechenden Verdünnungsmittel vor und tropft unter Kühlung der Reaktionsmischung das 1,2,4-Triazol-1-ylmethylketon der Formel (III) in einem Lösungsmittel gelöst zu. Nach beendeter Zugabe erwärmt man die Mischung zur Vervollständigung der Umsetzung auf die erforderliche Temperatur. Zur Aufarbeitung und Isolierung der Reaktionsprodukte der Formel (I) versetzt man das Reaktionsgemisch mit Wasser, extrahiert mit einem organischen Lösungsmittel und destilliert dieses anschließend gegebenenfalls unter vermindertem Druck ab.

Zur Herstellung von pflanzen-physiologisch verträglichen Säureadditionssalzen der Verbindungen der allgemeinen Formel (I) kommen vorzugsweise folgende Säuren infrage: Halogenwasserstoffsäuren, wie z. B. Chlorwasserstoffsäure und Bromwasserstoffsäure, insbesondere Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, sowie Sulfonsäuren, wie z. B. p-Toluolsulfonsäure und 1,5-Naphthalin-disulfonsäure.

Die Säureadditionssalze der Verbindungen der allgemeinen Formel (I) können in einfacher Weise nach üblichen Salzbildungsmethoden, z. B. durch Lösen einer Verbindung der allgemeinen Formel (I) in einem geeigneten organischen Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. Chlorwasserstoffsäureund Bromwasserstoffsäure, ferner Phosphorsäure, Salpetersäure und Schwefelsäure.

Die Metallsalz-Komplexe von Verbindungen der allgemeinen Formel (I) können in einfacher Weise nach üblichen Verfahren erhalten werden, so z. B. durch Lösen des Metallsalzes in Alkohol, wie z. B. Ethanol und Hinzufügen zur Verbindung der allgemeinen Formel (I). Man kann die Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkatoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis) oder gegen den Erreger der Netzfleckenkrankheit der Gerste (Pyrenophora teres) sowie zur Bekämpfung von Obst- und Gemüsekrankheiten, wie beispielsweise gegen den Erreger des Apfelschorfes (Venturia inaequalis) oder gegen den Erreger des Gurkenmehltaus (Sphaerotheca fuliginea) eingesetzt werden. Die erfindungsgemäßen Wirkstoffe zeichnen sich durch eine hervorragende Breitenwirkung aus.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole,

Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittelals Hilfs lösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Ärosol-Treibgase, wie Halogenkohlenwasserstoffe sowie Butan, Propan, Stickstoff und Kohlendioxid als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier-und/oder schaumerzeugende Mittel kommen in Frage: z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxld, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

**Herstellungsbeispiele:**

**Beispiel 1:**

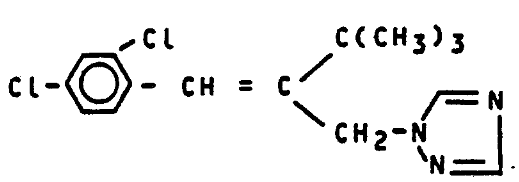

(Verfahren a)

Eine Mischung aus 20 g (0,62 Mol) 2-Brommethyl-1-(2,4-dichlorphenyl)-3,3-dimethyl-1-buten und 8,6g (0,125 Mol) Triazol in 80 ml absolutem Acetonitril wird 16 Stunden bei 40° C gerührt. Zur Aufarbeitung filtriert man die Reaktionsmischung, engt das Filtrat im Vakuum ein und nimmt den Rückstand in Dichlormethan auf. Man wäscht 2 mal mit Wasser, trocknet über Natriumsulfat und engt im Vakuum ein. Der zurückbleibende Sirup wird chromatographisch (Kieselgel) gereinigt und aus Petrolether kristallisiert. Man erhält 4,6 g (24 % der Theorie) an 1-(2,4-Dichlorphenyl)-3,3-dimethyl-2-(1,2,4-triazol-1-ylmethyl)-1-buten vom Schmelzpunkt 78° C.

<u>Herstellung der Ausgangsverbindung</u>:

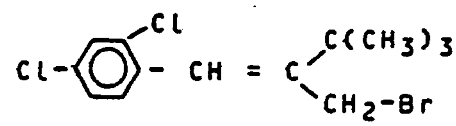

(II-1)

Eine Mischung aus 24,3 g (0,1 Mol) 1-(2,4-Dichlorphenyl)-2,3,3-trimethyl-1-buten, 17,8 g (0,1 Mol) N-Bromsuccinimid und einer Spatelspitze Azodiisobutyrodinitril in 100 ml absolutem 1,1,2,2-Tetrachlorethan wird über Nacht am Rückfluß gekocht. Zur Aufarbeitung engt man im Vakuum ein, nimmt den Rückstand in Tetrachlorkohlenstoff auf, filtriert und destilliert das Filtrat. Man erhält 20 g (62,1 % der Theorie) an 2-Brommethyl-1-(2,4-dichlorphenyl)-3,3-dimethyl-1-buten vom Siedepunkt 108° C bei 0,13 mbar.

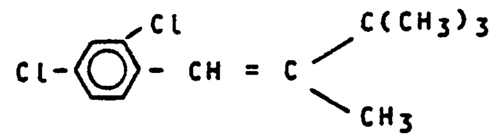

29,7 g (0,1 Mol) 2,4-Dichlorbenzylphosphonsäurediethylester, 10 g (0,1 Mol) Pinakolon, 12 g (0,1 Mol) Kalium-t-butylat und 300 ml absolutes Toluol werden in einer Stickstoffatmosphäre während 20 Stunden zum Sieden erhitzt. Die Lösung wird abgekühlt, 2 mal mit jeweils 80 ml Wasser gewaschen, über Natriumsulfat getrocknet und im Vakuum vom Lösungsmittel befreit. Nach Destillation des Rückstandes erhält man 15 g (69,7 % der Theorie) an 1-(2,4-Dichlorphenyl)-2,3,3-trimethyl-1-buten vom Siedepunkt 85° C bei 0,13 mbar.

**Beispiel 2:**

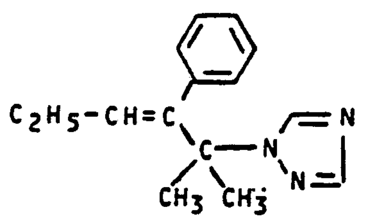

(Verfahren b)

Zu einer Suspension von 36,6 g (0,1 Mol) Triphenyl-n-propyl-phosphoniumbromid in 150 ml absolute, Tetrahydrofuran tropft man bei 0° C unter Stickstoffatmosphäre 61 ml (0,1 Mol) 15 %-ige n-Butyllithium-Lösung in Hexan und rührt 30 Minuten bei 0° C nach. Danach wird eine Suspension von 21,5 g (0,1 Mol) α-(1,2,4-Triazol-1-yl)-isobutyrophenon in 150 ml absolutem Tetrahydrofuran schnell zugetropft und nach beendeter Zugabe noch 3,5 Stunden bei Raumtemperatur nachgerührt. Danach erwärmt man für eine halbe Stunde auf 40°C, entfernt das Lösungsmittel im Vakuum, nimmt den Rückstand in Wasser auf und extrahiert mehrfach mit Chloroform. Die vereinigten organischen Phasen werden über Natriumsulfat getrocknet und im Vakuum eingedampft. Der Rückstand wird in Petrolether aufgenommen, zum Sieden erhitzt und filtriert.

Aus dem Filtrat erhält man nach Entfernen des Lösungsmittels im Vakuum durch Destillation 12,8 g (53 % der Theorie) an 2-Methyl-3-phenyl-2-(1,2,4-triazol-1-yl)-hex-3-en vom Siedepunkt 98° C bis 100° C bei 0,13 mbar.

Herstellung des Ausgangsstoffes:

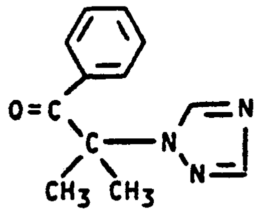

14 g (0,2 Mol) Triazol und 27 g (0,2 Mol) Kaliumcarbonat werden in 100 ml Acetonitril bei 75°C mit 45,4 g (0,2 Mol) α-Bromisobutyrophenon und 1 g Kaliumjodid versetzt. Nach beendeter Zugabe rührt man weitere 8 Stunden bei 75°C, filtriert und wäscht mit Acetonitril nach. Das Filtrat wird eingeengt, der Rückstand in Chloroform aufgenommen, zweimal mit Wasser gewaschen, über Natriumsulfat getrocknet und im Vakuum vom Lösungsmittel befreit. Der Rückstand wird aus Essigester/Petrolether umkristallisiert. Man erhält 12,1 g (28 % der Theorie) an α-(1,2,4-Triazol-1-yl)-isobutyrophenon vom Schmelzpunkt 134°C bis 135°C.

**Beispiel 3:**

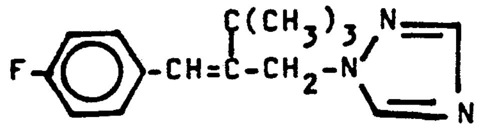

Öl

**Beispiel 4:**

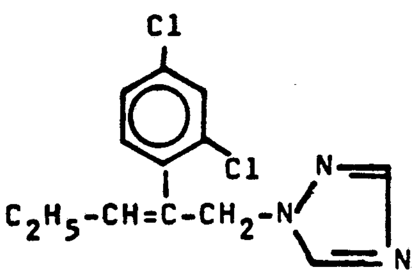

Öl

**Beispiel 5:**

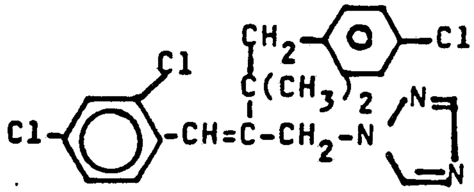

Fp. 118°C

**Anwendungsbeispiele:**

In den nachfolgenden Anwendungsbeispielen werden die nachstehend aufgeführten Verbindungen als Vergleichssubstanzen eingesetzt:

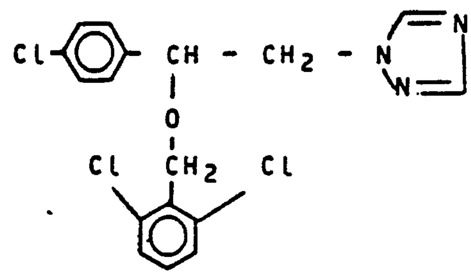

(A)

1-(4-Chlorphenyl)-1-(2,6-dichlorbenzyloxy)-2-(1,2,4-triazol-1-yl)-ethan

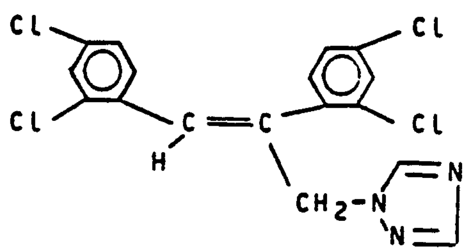

(B)

cis-1,2-Bis-(2,4-dichlorphenyl)-3-(1,2,4-triazol-1-yl)-1-propen

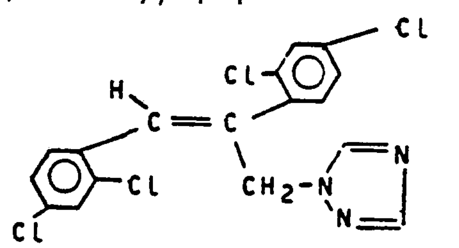

(C)

trans-1,2-Bis-(2,4-dichlorphenyl)-3-(1,2,4-triazol-1-yl)-1-propen

**Beispiel A**

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1.

**Beispiel B**

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Täge nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1.

**Beispiel C**

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1.

**Patentansprüche**

1. 3-(1,2,4-Triazol-1-yl)-1-propene der Formel

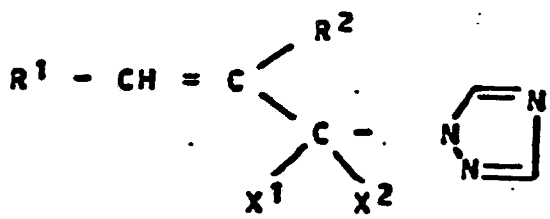

(I)

in welcher

a) entweder

$X^1$ und $X^2$ für Methyl stehen,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-,

i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für Methyl Ethyl n- und i-Propyl n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$ - C- Rest, für jeweils

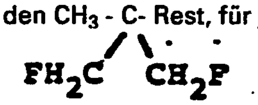

gegebenenfalls ein- bis dreifach, gleich oder verschieden substituiertes Phenyl oder Benzyl oder für einen im Phenylkern ein- bis dreifach, gleich oder verschieden substituierten

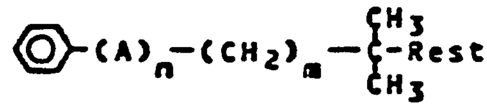

steht, wobei als Phenylsubstituenten jeweils die bei $R^1$ genannten infrage kommen; wobei

A für Sauerstoff oder Schwefel steht,

m für eine Zahl 0,1 oder 2 steht, und

n für eine Zahl von 0 oder 1 steht,

jedoch mit der Maßgabe, daß $R^1$ und $R^2$ nicht gleichzeitig für gegebenenfalls substuiertes Phenyl stehen dürfen,

oder

b) $X^1$ für Wasserstoff steht,

$X^2$ für Methyl steht,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-, i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für Methyl, Ethyl n- und i-Propyl n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$ - C- Rest, für jeweils

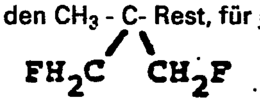

gegebenenfalls ein- bis dreifach, gleich oder verschieden substituiertes Phenyl oder Benzyl oder für einen im Phenylkern ein- bis dreifach, gleich oder verschieden substituierten

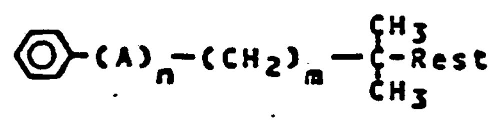

steht, wobei als Phenylsubstituenten jeweils die bei $R^1$ genannten infrage kommen; wobei

A für Sauerstoff oder Schwefel steht,

m für eine Zahl 0,1 oder 2 steht, und

n für eine Zahl von 0 oder 1 steht,

jedoch mit der Maßgabe, daß $R^1$ und $R^2$ nicht gleichzeitig für gegebenenfalls substuiertes Phenyl stehen dürfen,

oder

c) $X^1$ und $X^2$ für Wasserstoff stehen,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-, i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für n- und i-Pentyl n- und i-Hexyl, n- und i-Heptyl, Allyl, Propenyl, Butenyl, für jeweils gegebenenfalls

einfach oder mehrfach durch Methyl substituiertes Cyclopentyl, Cyclohexyl, Cyclohexenyl oder Cycloheptenyl steht oder für den Rest

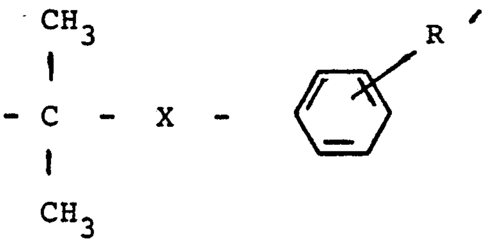

steht wobei

R' für Wasserstoff, Hydroxy, Fluor, Chlor, Brom, Cyano, Nitro, Methyl, Methoxy, Methylthio Trifluormethyl Trifluormethoxy, Trifluormethylthio, Ethyl, Ethoxy, Ethylthio, n- und i-Propyl, Isopropyloxy, n-, i-, s- und t-Butyl oder Cyclohexyl steht und

X für $CH_2$, Sauerstoff, Schwefel steht,

sowie deren pflanzenverträgliche Säureadditions-Salze und Metallsalz-Komplexe.

2. Verfahren zur Herstellung von 3-(1,2,4-Triazol-1-yl)-1-propenen der Formel

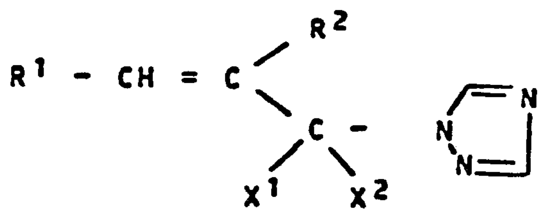

(I)

in welcher

a) entweder

$X^1$ und $X^2$ für Methyl stehen,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-, i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für Methyl Ethyl n- und i-Propyl n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$ - C- Rest, für jeweils

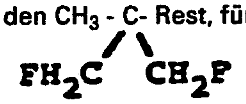

gegebenenfalls ein- bis dreifach, gleich oder verschieden substituiertes Phenyl oder Benzyl oder für einen im Phenylkern ein- bis dreifach, gleich oder verschieden substituierten

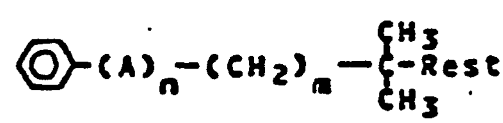

steht, wobei als Phenylsubstituenten jeweils die bei $R^1$ genannten infrage kommen; wobei

A für Sauerstoff oder Schwefel steht,

m für eine Zahl 0,1 oder 2 steht, und

n für eine Zahl von 0 oder 1 steht, jedoch mit der Maßgabe, daß $R^1$ und $R^2$ nicht gleichzeitig für gegebenenfalls substuiertes Phenyl stehen dürfen,

oder

b) $X^1$ für Wasserstoff steht,

$X^2$ für Methyl steht,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder

verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-, i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für Methyl Ethyl n- und i-Propyl n-, i-, s- und t-Butyl, n- und i-Pentyl, n- und i-Hexyl, n- und i-Heptyl steht, für Allyl, Propenyl, Butenyl, für jeweils gegebenenfalls einfach oder mehrfach durch Methyl substituiertes Cyclopentyl, Cyclohexyl, Cyclohexenyl oder Cycloheptyl steht, für den $CH_3$ - C- Rest, für jeweils

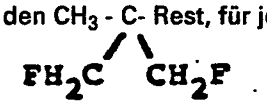

gegebenenfalls ein- bis dreifach, gleich oder verschieden substituiertes Phenyl oder Benzyl oder für einen im Phenylkern ein- bis dreifach, gleich oder verschieden substituierten

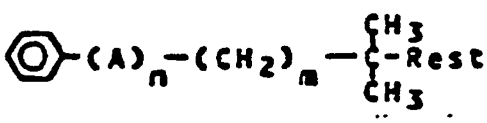

steht, wobei als Phenylsubstituenten jeweils die bei $R^1$ genannten infrage kommen; wobei

A für Sauerstoff oder Schwefel steht,

m für eine Zahl 0,1 oder 2 steht, und

n für eine Zahl von 0 oder 1 steht,

jedoch mit der Maßgabe, daß $R^1$ und $R^2$ nicht gleichzeitig für gegebenenfalls substuiertes Phenyl stehen dürfen,

oder

c) $X^1$ und $X^2$ für Wasserstoff stehen,

$R^1$ für Methyl, Ethyl, n- und i-Propyl, n-, i-, s- und t-Butyl, n-Pentyl, neo-Pentyl, n- und i-Hexyl-, für Allyl, Butenyl, für jeweils gegebenenfalls einfach oder mehrfach durch Methyl substituiertes Cyclopentyl, Cyclohexyl, Cyclohexenyl oder Cycloheptyl steht, oder für jeweils gegebenenfalls ein- bis dreifach, gleich oder verschieden substituiertes Phenyl-oder Benzyl steht, wobei als Substituenten zu nennen sind:

Hydroxy, Fluor, Chlor, Brom, Cyano, Nitro, Methyl, Methoxy, Methylthio, Methoxycarbonyl, Trifluormethyl, Trifluormethoxy, Trifluormethylthio, Ethyl, Ethoxy, Ethylthio, Ethoxycarbonyl, n- und i-Propyl, Isopropyloxy, n-, i-, s- und t-Butyl, Cyclohexyl, Dimethylamino, Diethylamino, Acetyloxy, Acetamido, N-Methylacetamido sowie durch Fluor, Chlor oder Methyl, ein- bis dreifach, gleich oder verschieden substituiertes Phenyl oder Phenoxy, und

$R^2$ für n- und i-Pentyl n- und i-Hexyl, n- und i-Heptyl, Allyl, Propenyl, Butenyl, für jeweils gegebenenfalls einfach oder mehrfach durch Methyl substituiertes Cyclopentyl, Cyclohexyl, Cyclohexenyl oder Cycloheptenyl steht oder für den Rest

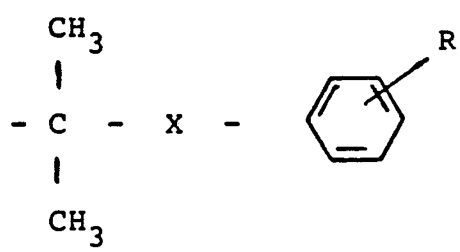

steht wobei

R' für Wasserstoff, Hydroxy, Fluor, Chlor, Brom, Cyano, Nitro, Methyl, Methoxy, Methylthio Trifluormethyl Trifluormethoxy, Trifluormethylthio, Ethyl, Ethoxy, Ethylthio, n- und i-Propyl, Isopropyloxy, n-, i-, s- und t-Butyl oder Cyclohexyl steht und

X für $CH_2$, Sauerstoff, Schwefel steht,

sowie von deren pflanzenverträglichen Säureadditions-Salzen und Metallsalz-Komplexen, dadurch gekennzeichnet, daß man

a) 3-Halogen- bzw. 3-Sulfonyloxy-1-propene der Formel

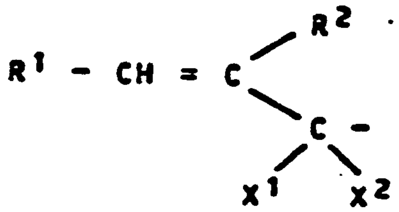

(II)

in welcher
$R^1$, $R^2$, $X^1$ und $X^2$ die oben engegebene Bedeutung haben und
Y für Chlor Brom Methansulfonyloxy oder p-Toluolsulfonyloxy steht,
mit 1,2,4-Triazol gegebenenfalls in Gegenwart eines Verdünnungsmittels und gegebenenfalls in Gegenwart eines Säurebindemittels umsetzt; oder daß man
b) 1,2,4-Triazol-1-ylmethylketone der Formel

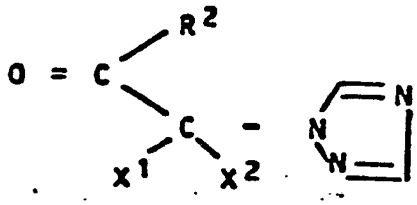

(III)

in welcher
$R^2$, $X^1$ und $X^2$ die oben angegebene Bedeutung haben mit Phosphonsäureestern bzw. Phosphinoxiden der Formel

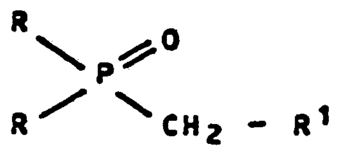

(IVa)

in welcher
$R^1$ die oben angegeben Bedeutung hat und
R für Methoxy, Ethoxy oder Phenyl steht,
oder alternativ mit Triphenylalkylidenphosphoranen der Foreml

$(C_6H_5)_3P = CH - R^1$ (IVb)

in welcher
$R^1$ die oben angegebene Bedeutung hat,
in Gegenwart eines Verdünnungsmittels und in Gegenwart einer Base im Sinne einer "Wittig-Olefinierung" umsetzt, und gegebenenfalls noch an die so erhaltenen Verbindungen der Formel (I) eine Säure oder ein Metallsalz addiert.

3. Fungizide Mittel, gekennzeichnet durch einen Gehalt an mindestens einem 3-(1,2,4-Triazol-1-yl)-1-propen der Formel (I) in Anspruch 1 bzw. einem pflanzenverträglichen Säureadditions-Salz oder Metallsalz-Komplex eines 3-(1,2,4-Triazol-1-yl)-1-propens der Formel (I).

4. Verfahren zur Bekämpfung von Pilzen, dadurch gekennzeichnet, daß man 3-(1,2,4-Triazol-1-yl)-1-propene der Formel (I) gemäß Anspruch 1 bzw. deren pflanzenverträgliche Säureadditions-Salze oder Metallsalz-Komplexe auf Pilze oder ihren Lebensraum einwirken läßt.

5. Verwendung von 3-(1,2,4-Triazol-1-yl)-1-propenen der Formel (I) gemäß Anspruch 1 bzw. von deren pflanzenverträglichen Säureadditions-Salzen und Metallsalz-Komplexen zur Bekämpfung von Pilzen.

6. Verwendung von 3-(1,2,4-Triazol-1-yl)-1-propenen der Formel (1) gemäß Anspruch 1 bzw. von deren pflanzenverträglichen Säureadditions-Salzen oder Metallsalz-Komplexen als Pflanzenschutzmittel.

7. Verfahren zur Herstellung von fungiziden Mitteln, dadurch gekennzeichnet, daß man 3-(1,2,4-Triazol-1-yl)-1-propene der Formel (1) gemäß Anspruch 1 bzw. deren pflanzenverträgliche Säureadditions-Salze oder Metallsalz-Komplexe mit Streckmitteln und/oder oberflächenaktiven Stoffen vermischt.

**Claims**

1. 3-(1,2,4-Triazol-1-yl)-prop-1-enes of the formula

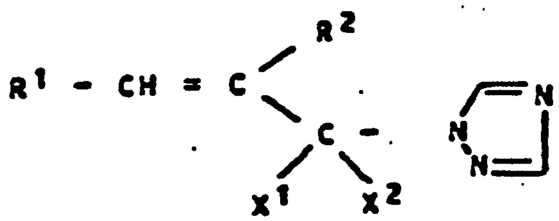

(I)

in which

a) either

$X^1$ and $X^2$ represent methyl,

$R^1$ represents methyl, ethyl, n- or i-propyl, n-, i-, s- or t-butyl, n-pentyl, neo-pentyl, n- or i-hexyl-, allyl, butenyl, or cyclopentyl, cyclohexyl, cyclohexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro, methyl, methoxy, methylthio, methoxycarbonyl, trifluoromethyl, trifluoromethoxy, trifluoromethylthio, ethyl, ethoxy, ethylthio, ethoxycarbonyl, n- or i-propyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents methyl, ethyl, n- or i-propyl, n-, i-, s- or t-butyl, n- or i-pentyl, n- or ihexyl, n- or i-heptyl, allyl, propenyl, butenyl, or cyclopentyl, cyclohexyl, cyclohexenyl or cycloheptyl, in each case optionally mono- or poly-substituted by methyl, the $CH_3$ - C-

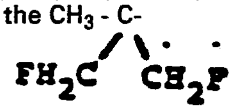

radical, phenyl or benzyl, in each case optionally mono-, di- or tri-substituted by identical or different substituents, or a 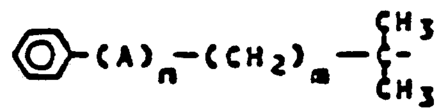 radical, which is mono-, di- or tri-substituted by identical or different substituents in the phenyl nucleus, possible substituents on the phenyl in each case being those mentioned for $R^1$:

wherein

A represents oxygen or sulphur,

m represents the number 0, 1 or 2 and

n represents the number 0 or 1,

but with the proviso that $R^1$ and $R^2$ may not simultaneously represent optionally substituted phenyl, or

b) $X^1$ represents hydrogen,

$X^2$ represents methyl,

$R^1$ represents methyl, ethyl, n- or i-propyl, n-, i-, s- or t-butyl, n-pentyl, neo-pentyl, nor i-hexyl-, allyl, butenyl, or cyclopentyl, cyclohexyl, cyclohexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro, methyl, methoxy, methylthio, methoxycarbonyl, trifluoromethyl, trifluoromethoxy, trifluoromethylthio, ethyl, ethoxy, ethylthio, ethoxycarbonyl, n- or ipropyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents methyl, ethyl, n- or i-propyl, n-, i-, s- or t-butyl, n- or i-pentyl, n- or i-hexyl, n- or i-heptyl, allyl, propenyl, butenyl, or cyclopentyl, cyclohexyl, cyclohexenyl or cycloheptyl, in each case optionally mono- or poly-substituted by methyl, the $CH_3$ - C-

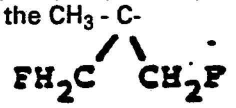

radical, phenyl or benzyl, in each case optionally mono-, di- or tri-substituted by identical or different substituents, or a 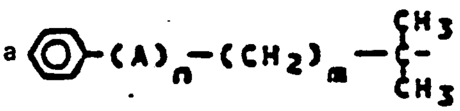 radical, which is mono-, di- or tri-substituted by identical or different substituents in the phenyl nucleus, possible substituents on the phenyl in each case being those mentioned for $R^1$:

wherein

A represents oxygen or sulphur,

m represents the number 0,1 or 2 and

n represents the number 0 or 1,

but with the proviso that $R^1$ and $R^2$ may not simultaneously represent optionally substituted phenyl, or

c) $X^1$ and $X^2$ represent hydrogen,

$R^1$ represents methyl, ethyl, n- or i-propyl, n-, i-, s- or t-butyl, n-pentyl, neo-pentyl, n- or i-hexyl-, allyl, butenyl, or cyclopentyl, cyclohexyl, cyclohexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro, methyl, methoxy, methylthio, methoxycarbonyl, trifluoromethyl, trifluoromethoxy, trifluoromethylthio, ethyl, ethoxy, ethylthio, ethoxycarbonyl, n- or i-propyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents n- or i-pentyl, n- or i-hexyl, nor i-heptyl, allyl, propenyl, butenyl, or cyclopentyl, cyclohexyl, cyclohexenyl or cycloheptenyl, in each case optionally mono- or poly-substituted by methyl, or the

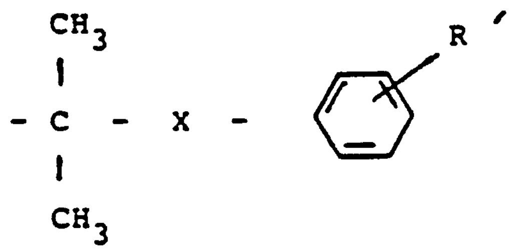

radical,

wherein

R' represents hydrogen, hydroxyl, fluorine, chlorine, bromine, cyano, nitro, methyl, methoxy, methylthio, trifluoromethyl, trifluoromethoxy, trifluoromethylthio, ethyl, ethoxy, ethylthio, n- or i-propyl, isopropyloxy, n-, i-, s- or t-butyl or cyclohexyl, and

X represents $CH_2$, oxygen or sulphur, and acid addition salts and metal salt complexes thereof which are tolerated by plants.

2. Process for the preparation of 3-(1,2,4-triazol-1-yl)-prop-1-enes of the formula

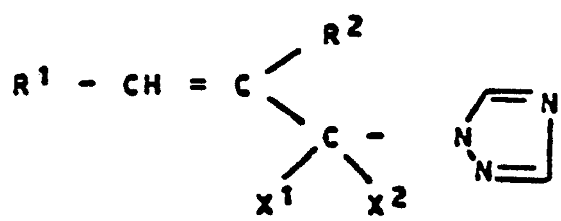

(I)

in which

a) either

$X^1$ and $X^2$ represent methyl,

$R^1$ represents methyl ethyl, n- or i-propyl, n-, i-, s- or t-butyl, n-pentyl, neo-pentyl, n- or i-hexyl-, allyl, butenyl, or cyclopentyl, cyclohexyl, cyclohexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro,

methyl, methoxy, methylthio, methoxycarbonyl, trifluoromethyl, trifluoromethoxy, trifluoromethylthio, ethyl, ethoxy, ethylthio, ethoxycarbonyl, n- or i-propyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents methyl, ethyl, n- or i-propyl, n-, i-, s- or t-butyl, n- or i-pentyl, n- or i-hexyl, n- or i-heptyl, allyl, propenyl, butenyl, or cyclopentyl, cyclohexyl, cyclohexenyl or cycloheptyl, in each case optionally mono- or poly-substituted by methyl, the $CH_3$ - C-

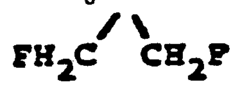

radical, phenyl or benzyl, in each case optionally mono-, di- or tri-substituted by identical or different substituents, or a 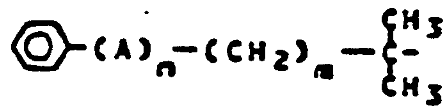 radical, which is mono-, di- or tri-substituted by identical or different substituents in the phenyl nucleus, possible substituents on the phenyl in each case being those mentioned for $R^1$:

wherein

A represents oxygen or sulphur,

m represents the number 0, 1 or 2 and

n represents the number 0 or 1,

but with the proviso that $R^1$ and $R^2$ may not simultaneously represent optionally substituted phenyl, or

b) $X^1$ represents hydrogen,

$X^2$ represents methyl,

$R^1$ represents methyl, ethyl, n- or i-propyl, n-, i-, s- or t-butyl, n-pentyl, neo-pentyl, n-or i-hexyl-, allyl, butenyl, or cyclopentyl, cyclohexyl, cyclonexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro, methyl, methoxy, methylthio, methoxycarbonyl, trifluoromethyl, trifluoromethoxy, trifluoromethylthio, ethyl, ethoxy, ethylthio, ethoxycarbonyl, n- or i-propyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents methyl, ethyl, n- or i-propyl, n-, i-, s- or t-butyl, n- or i-pentyl, n- or i-hexyl, n- or i-heptyl, allyl, propenyl, butenyl, or cyclopentyl, cyclohexyl, cyclohexenyl or cycloheptyl, in each case optionally mono- or poly-substituted by methyl, the $CH_3$ - C-

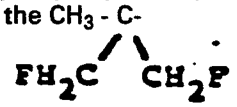

radical, phenyl or benzyl, in each case optionally mono-, di- or tri-substituted by identical or different substituents, or a 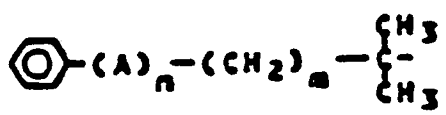 radical, which is mono-, di- or tri-substituted by identical or different substituents in the phenyl nucleus, possible substituents on the phenyl in each case being those mentioned for $R^1$:

wherein

A represents oxygen or sulphur,

m represents the number 0, 1 or 2 and

n represents the number 0 or 1,

but with the proviso that $R^1$ and $R^2$ may not simultaneously represent optionally substituted phenyl, or

c) $X^1$ and $X^2$ represent hydrogen,

$R^1$ represents methyl, ethyl, n- or i-propyl, n-, i-, s- or t-butyl, n-pentyl, neo-pentyl, n- or i-hexyl-, allyl, butenyl, or cyclopentyl, cyclohexyl, cyclohexenyl or cycloheptyl, in each case optionally mono- or poly-substituted by methyl, or phenyl or benzyl, in each case optionally mono-, di- or tri-substituted by identical or different substituents, substituents which may be mentioned being: hydroxyl, fluorine, chlorine, bromine, cyano, nitro, methyl, methoxy, methylthio, methoxycarbonyl, trifluoromethyl, trifluoromethoxy, trifluoromethylthio, ethyl, ethoxy, ethylthio, ethoxycarbonyl, n- or i-propyl, isopropyloxy, n-, i-, s- or t-butyl, cyclohexyl, dimethylamino, diethylamino, acetyloxy, acetamido, N-methylacetamido and phenyl or phenoxy which is mono-, di- or tri-substituted by identical or different fluorine, chlorine or methyl substituents, and

$R^2$ represents n- or i-pentyl, n- or i-hexyl, n- or i-heptyl, allyl, propenyl, butenyl, or cyclopentyl, cyclohexyl, cyclohexenyl or cycloheptenyl, in each case optionally mono- or poly-substituted by methyl, or the

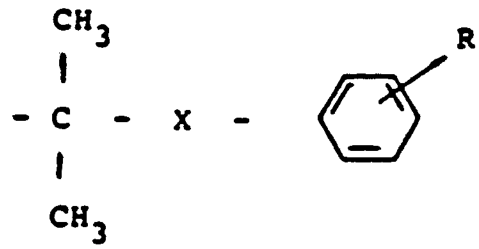

radical,

wherein

R' represents hydrogen hydroxyl fluorine, chlorine, bromine, cyano nitro methyl methoxy, methylthio, trifluoromethyl, trifluoromethoxy, trifluoromethylthio, ethyl, ethoxy, ethylthio, n- or i-propyl, isopropyloxy, n-, i-, s- or t-butyl or cyclohexyl, and

X represents $CH_2$, oxygen or sulphur, and of acid addition salts and metal salt complexes thereof which are tolerated by plants,

characterised in that

a) 3-halogeno- or 3-sulphonyloxy-prop-1-enes of the formula

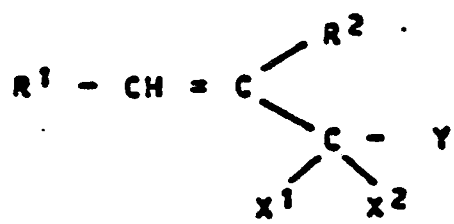

(II)

in which

$R^1$, $R^2$, $X^1$ and $X^2$ have the abovementioned meaning and

Y represents chlorine, bromine, methanesulphonyloxy or p-toluenesulphonyloxy

are reacted with 1,2,4-triazole, if appropriate in the presence of a diluent and if appropriate in the presence of an acid-binding agent; or in which

b) 1,2,4-triazol-1-ylmethyl ketones of the formula

(III)

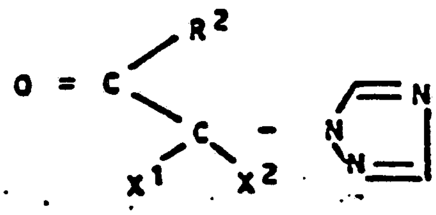

in which

$R^1$, $X^1$ and $X^2$ have the abovementioned meaning, are reacted with phosphonic acid esters or phosphine oxides of the formula

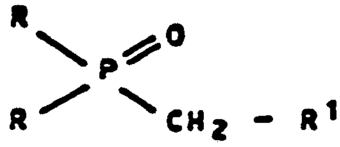

(IVa)

in which

$R^1$ has the abovementioned meaning and

R represents methoxy, ethoxy or phenyl,

or, alternatively, with triphenylalkylidene-phosphoranes of the formula

$(C_6H_5)_3P = CH - R^1$ (IVb)

in which

$R^1$ has the abovementioned meaning, in the presence of a diluent and in the presence of a base, in the sense

of a "Witting olefination", and, if appropriate, an acid or a metal salt is also added onto the compounds of the formula (I) thus obtained.

3. Fungicidal agents, characterised in that they contain at least one 3-(1,2,4-triazol-1-yl)-prop-1-ene of the formula (I) in claim 1 or an acid addition salt or metal salt complex of a 3-(1,2,4-triazol-1-yl)-prop-1-ene of the formula (I) which is tolerated by plants.

4. Method of combating fungi, characterised in that 3-(1,2,4-triazol-1-yl)-prop-1-enes of the formula (I) according to Claim 1 or the acid addition salts or metal salt complexes thereof which are tolerated by plants are allowed to act on fungi or their environment.

5. Use of 3-(1,2,4-triazol-1-yl)-prop-1-enes of the formula (I) according to Claim 1 or the acid addition salts or metal salt complexes thereof which are tolerated by plants for combating fungi.

6. Use of 3-(1,2,4-triazol-1-yl)-prop-1-enes of the formula (I) according to Claim 1 or the acid addition salts or metal salt complexes thereof which are tolerated by plants as plant protection agents.

7. Process for the preparation of fungicidal agents, characterised in that 3-(1,2,4-triazol-1-yl)-prop-1-enes of the formula (I) according to Claim 1 or the acid addition salts or metal salt complexes thereof which are tolerated by plants are mixed with extenders and/or surface-active agents.

**Revendications**

1. 3-(1,2,4-triazole-1-yl)-1-propènes de formule

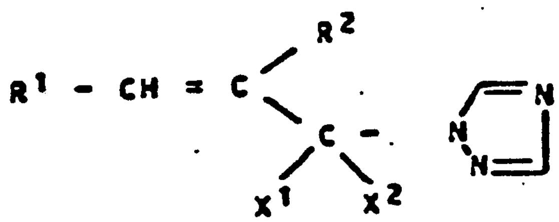

. (I)

dans laquelle

a)

$X^1$ et $X^2$ représentent un groupe méthyle,

$R^1$ est un groupe méthyle éthyle, n-propyle, isopropyle, n-butyle, isobutyle, sec.-butyle, tertio-butyle, n-pentyle, néopentyle, n-hexyle, isohexyle, un groupe allyle, butényle, un groupe cyclopentyle, cyclohexyle, cyclohexényle ou cycloheptyle substitués chacun, le cas échéant, avec un ou plusieurs groupes méthyle, ou un groupe phényle ou benzyle portant chacun, le cas échéant, un à trois substituants identiques ou différents, et on peut mentionner alors comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec.-butyle, tertio-butyle, cyclohexyle, diméthylamino, diéthylamino, acétyloxy, acétamido, N-méthylacétamido, ainsi que phényle ou phénoxy portant éventuellement un à trois substituants fluoro, chloro ou méthyle identiques ou différents, et

$R^2$ est un groupe méthyle éthyle n-propyle, isopropyle, n-butyle isobutyle sec.-butyle, tertio-butyle, n-pentyle, isopentyle, n-hexyle, isohexyle, n-heptyle et isoheptyle, un groupe allyle, propényle, butényle, un groupe cyclopentyle, cyclohexyle, cyclohexényle ou cycloheptyle portant chacun, le cas échéant, un ou plusieurs substituants méthyle, le reste

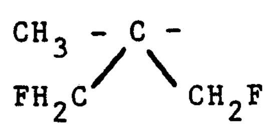

un reste phényle ou benzyle portant chacun éventuellement un à trois substituants identiques ou différents ou un reste

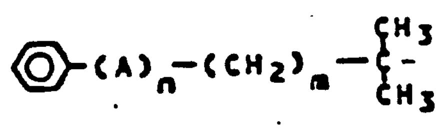

portant dans le noyau phényle un à trois substituants identiques ou différents, et on considère alors comme substituants du groupe phényle dans chaque cas ceux qui sont mentionnés pour $R^1$;

A représente l'oxygène ou le soufre,

m a la valeur 0, 1 ou 2, et

n a la valeur 0 ou 1,

mais à condition que $R^1$ et $R^2$ ne puissent pas représenter en même temps un groupe phényle éventuellement substitué, ou bien

b)

$X^1$ représente l'hydrogène

$X^2$ est un groupe méthyle,

$R^1$ est un groupe méthyle, éthyle, n-propyle, isopropyle, n-butyle, isobutyle, sec.-butyle, tertio-butyle, n-pentyle, néopentyle, n-hexyle et iso-hexyle, un groupe allyle, butényle, un groupe cyclopentyle, cyclohexyle, cyclohexényle ou cycloheptyle portant chacun, le cas échéant, un ou plusieurs substituants méthyle ou un groupe phényle ou benzyle portant chacun le cas échéant, un à trois substituants identiques ou différents, et on peut alors mentionner comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec.-butyle, tertio-butyle, cyclohexyle, diméthylamino, diéthylamino, acétyloxy, acétamido, N-méthylacétamido ainsi qu'un groupe phényle ou phénoxy portant un à trois substituants fluoro, chloro ou méthyle identiques ou différents, et

$R^2$ est un groupe méthyle, éthyle, n-propyle, isopropyle, n-butyle, isobutyle, sec.-butyle, tertio-butyle, n-pentyle, isopentyle, n-hexyle, isohexyle, n-heptyle et isoheptyle, un groupe allyle, propényle, butényle, un groupe cyclopentyle, cyclohexyle, cyclohexényle ou cycloheptyle portant chacun, le cas échéant, un ou plusieurs substituants méthyle, le reste

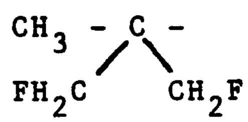

un reste phényle ou benzyle portant chacun, le cas échéant, un à trois substituants identiques ou différents, ou un reste

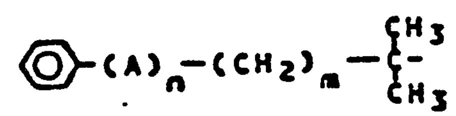

portant dans le noyau phényle un à trois substituants identiques ou différents, et on considère alors comme substituants du noyau phényle dans chaque cas les substituants mentionnés pour $R^1$;

A désigne l'oxygène ou le soufre,

m a la valeur 0, 1 ou 2, et

n a la valeur 0 ou 1,

mais à condition que $R^1$ et $R^2$ ne puissent pas représenter en même temps un groupe phényle éventuellement substitué,

ou bien

c) $X^1$ et $X^2$ représentent de l'hydrogène,

$R^1$ est un groupe méthyle, éthyle, n-propyle, isopropyle, n-butyle, isobutyle, sec.-butyle, tertio-butyle, n-pentyle, néopentyle, n-hexyle, isohexyle, un groupe allyle, butényle, un groupe cyclopentyle, cyclohexyle, cyclohexényle ou cycloheptyle portant chacun, le cas échéant, un ou plusieurs substituants méthyle, ou un groupe phényle ou benzyle portant chacun, le cas échéant, un à trois substituants identiques ou différents, et on peut alors mentionner comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec. -butyle, tertio-butyle, cyclohexyle, diméthylamino, diéthylamino, acétyloxy, acétamido, N-méthylacétamido, ainsi qu' un groupe phényle ou phénoxy portant un à trois substituants fluoro, chloro ou méthyle identiques ou différents, et

$R^2$ est un groupe n-pentyle, isopentyle, n-hexyle, isohexyle, n-heptyle, isoheptyle, allyle, propényle, butényle, un groupe cyclopentyle, cyclohexyle, cyclohexényle ou cycloheptényle portant chacun, le cas échéant, un ou plusieurs substituants méthyle, ou le reste

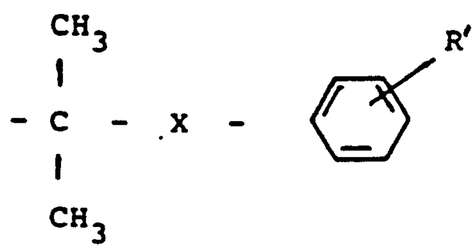

dans lequel

R' représente l'hydrogène, un groupe hydroxy, le fluor, le chlore, le brome, un groupe cyano, nitro, méthyle, méthoxy, méthylthio, trifluorométhyle, trifluorométhoxy, trifluorométhylthio, éthyle, éthoxy, éthylthio, n-propyle, isopropyle, isopropyloxy, n-butyle, isobutyle, sec.-butyle, tertio-butyle ou cyclohexyle et

X représente un groupe $CH_2$, l'oxygène ou le soufre, ainsi que leurs sels d'addition d'acides et leurs complexes de sels métalliques compatibles avec les plantes.

2. Procédé de production de 3-(1,2,4-triazole-1-yl)-1-propènes de formule

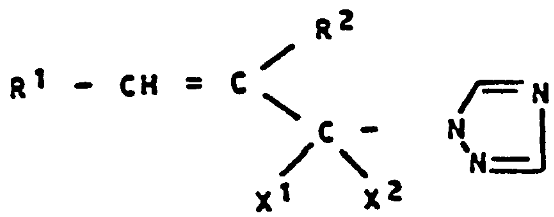

(I)

dans laquelle

a)

$X^1$ et $X^2$ représentent un groupe méthyle,

$R^1$ est un groupe méthyle, éthyle, n-propyle, isopropyle, n-butyle, isobutyle, sec.-butyle, tertio-butyle, n-pentyle, néopentyle, n-hexyle, isohexyle, un groupe allyle, butényle, un groupe cyclopentyle, cyclohexyle, cyclohexényle ou cycloheptyle substitués chacun, le cas échéant, avec un ou plusieurs groupes méthyle, ou un groupe phényle ou benzyle portant chacun, le cas échéant, un à trois substituants identiques ou différents, et on peut mentionner alors comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec.-butyle, tertio-butyle, cyclohexyle, diméthylamino, diéthylamino, acétyloxy, acétamido, N-méthylacétamido, ainsi que phényle ou phénoxy portant éventuellement un à trois substituants fluoro, chloro ou méthyle identiques ou différents, et

$R^2$ est un groupe méthyle, éthyle, n-propyle, isopropyle, n-butyle, isobutyle, sec. -butyle, tertio-butyle, n-pentyle, isopentyle, n-hexyle, isohexyle, n-heptyle et isoheptyle, un groupe allyle, propényle, butényle, un groupe cyclopentyle, cyclohexyle, cyclohexényle ou cycloheptyle portant chacun, le cas échéant, un ou plusieurs substituants méthyle, le reste

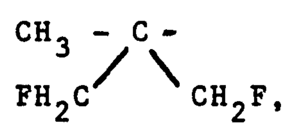

un reste phényle ou benzyle portant chacun éventuellement un à trois substituants identiques ou différents ou un reste

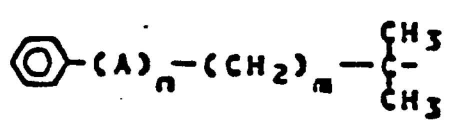

portant dans le noyau phényle un à trois substituants identiques ou différents, et on considère alors comme substituants du groupe phényle dans chaque cas ceux qui sont mentionnés pour $R^1$;

A représente l'oxygène ou le soufre,

m a la valeur 0, 1 ou 2, et

n a la valeur 0 ou 1, mais à condition que $R^1$ et $R^2$ ne puissent pas représenter en même temps un groupe phényle éventuellement substitué,

ou bien

b)

$X^1$ représente l'hdyrogène

$X^2$ est un groupe méthyle,

$R^1$ est un groupe méthyle, éthyle, n-propyle, isopropyle, n-butyle, isobutyle, sec.-butyle, tertio-butyle, n-pentyle, néopentyle, n-hexyle et isohexyle, un groupe allyle, buténylе, un groupe cyclopentyle, cyclohexyle, cyclohexényle ou cycloheptyle portant chacun le cas échéant, un ou plusieurs substituants méthyle, ou un groupe phényle ou benzyle portant chacun, le cas échéant, un à trois substituants identiques ou différents, et on peut alors mentionner comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec.-butyle, tertio-butyle, cyclohexyle, diméthylamino, diéthylamino, acétyloxy, acétamido, N-méthylacétamido ainsi qu'un groupe phényle ou phénoxy portant un à trois substituants fluoro, chloro ou méthyle, identiques ou différents, et

$R^2$ est un groupe méthyle, éthyle, n-propyle, isopropyle, n-butyle, isobutyle, sec.-butyle, tertio-butyle, n-pentyle, isopentyle, n-hexyle, isohexyle, n-heptyle et isoheptyle, un groupe allyle, propényle, butényle, un groupe cyclopentyle, cyclohexyle, cyclohexényle ou cycloheptyle portant chacun, le cas échéant, un ou plusieurs substituants méthyle, le reste

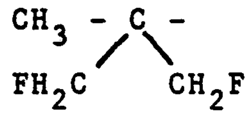

un reste phényle ou benzyle portant chacun, le cas échéant, un à trois substituants identiques ou différents, ou un reste

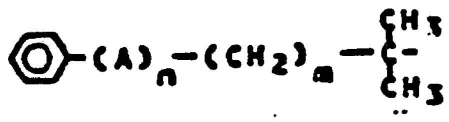

portant dans le noyau phényle un à trois substituants identiques ou différents, et on considère alors comme substituants du noyau phényle dans chaque cas les substituants mentionnés pour $R^1$;

A désigne l'oxygène ou le soufre,

$\underline{m}$ a la valeur 0, 1 ou 2, et

$\underline{n}$ a la valeur 0 ou 1,

mais à condition que $R^1$ et $R^2$ ne puissent pas représenter en même temps un groupe phényle éventuellement substitué,

ou bien

c) $X^1$ et $X^2$ représentent de l'hydrogène,

$R^1$ est un groupe méthyle, éthyle, n-propyle, isopropyle, n-butyle, isobutyle, sec.-butyle, tertio-butyle, n-pentyle, néopentyle, n-hexyle, isohexyle, un groupe allyle, butényle, un groupe cyclopentyle, cyclohexyle, cyclohexényle ou cycloheptyle portant chacun, le cas échéant, un ou plusieurs substituants méthyle, ou un groupe phényle ou benzyle portant chacun, le cas échéant, un à trois substituants identiques ou différents, et on peut alors mentionner comme substituants les substituants suivants: hydroxy, fluor, chlore, brome, cyano, nitro, méthyle, méthoxy, méthylthio, méthoxycarbonyle, trifluorométhyle, trifluorométhoxy, trifluorométhylthio, éthyle, éthoxy, éthylthio, éthoxycarbonyle, n-propyle, isopropyle, isopropyloxy, n-butyle, isobutyle, sec.-butyle, tertio-butyle, cyclohexyle, diméthylamino, diéthylamino, acétyloxy, acétamido, N-méthylacétamido, ainsi qu'un groupe phényle ou phénoxy portant un à trois substituants fluoro, chloro ou méthyle identiques ou différents, et

$R^2$ est un groupe n-pentyle, isopentyle, n-hexyle, isohexyle, n-heptyle, isoheptyle, allyle, propényle, butényle, un groupe cyclopentyle, cyclohexyle, cyclohexényle ou cycloheptényle portant chacun le cas échéant un ou plusieurs substituants méthyle, ou le reste

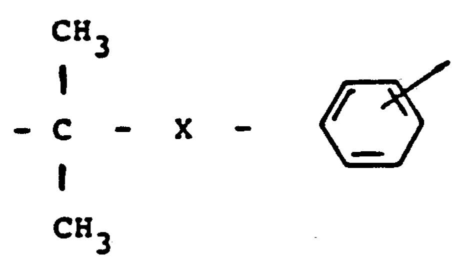

dans lequel

R' représente l'hydrogène, un groupe hydroxy, le fluor, le chlore, le brome, un groupe cyano, nitro, méthyle,

méthoxy, méthylthio, trifluorométhyle, trifluorométhoxy, trifluorométhylthio, éthyle, éthoxy, éthylthio, n-propyle, isopropyle, isopropyloxy, n-butyle, isobutyle, sec.-butyle, tertio-butyle ou cyclohexyle et

X représente un groupe $CH_2$, l'oxygène ou le soufre, ainsi que leurs sels d'addition d'acides et leurs complexes de sels métalliques compatibles avec les plantes, caractérisé en ce que:

a) on fait réagir des 3-halogéno- ou 3-sulfonyloxy -1-propènes de formule

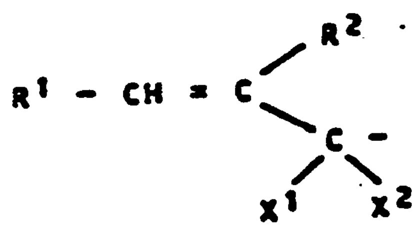

(II)

dans laquelle

$R^1$, $R^2$, $X^1$ et $X^2$ ont la définition indiquée ci-dessus et

Y représente le chlore, le brome, un groupe méthanesulfonyloxy ou p-toluènesulfonyloxy, avec le 1,2,4-triazole, le cas échéant en présence d'un diluant et en la présence éventuelle d'un accepteur d'acide; ou bien

b) on fait réagir des 1,2,4-triazole-1-ylméthylcétones de formule

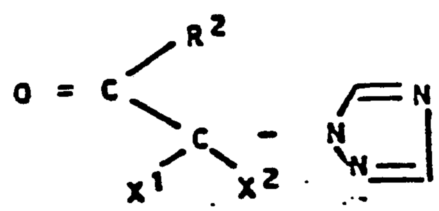

(III)

dans laquelle

$R^2$, $X^1$ et $X^2$ ont la définition indiquée ci-dessus, avec des esters d'acide phosphonique ou des oxydes de phosphines de formule

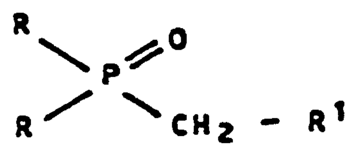

(IVa)

dans laquelle

$R^1$ a la définition indiquée ci-dessus et

$R^2$ est un groupe méthoxy, éthoxy ou phényle, ou bien à titre de variante avec des triphénylalkylidènephosphoranes de formule

$$(C_6H_5)_3P = CH - R^1 \qquad \text{(IVb)}$$

dans laquelle

$R^1$ a la définition indiquée ci-dessus, en présence d'un diluant et en présence d'une base, dans le sens d'une "oléfination de Wittig", et on additionne encore, le cas échéant, sur les composés de formule (I) ainsi obtenus un acide ou un sel métallique.

3. Compositions fongicides, caractérisées par une teneur en au moins un 3-(1,2,4-triazole-1-yl)-1-propène de formule (I) suivant la revendication 1 ou un sel d'addition d'acide ou un complexe de sel métallique compatible avec les plantes d'un 3-(1,2,4-triazole-1-yl)-1-propène de formule (I).

4. Procédé pour combattre des champignons, caractérisé en ce qu'on fait agir des 3-(1,2,4-triazole-1-yl)-1-propène de formule (I) suivant la revendication 1 ou leurs sels d'addition d'acides ou leurs complexes de sels métalliques compatibles avec les plantes sur des champignons ou sur leur milieu.

5. Utilisation de 3-(1,2,4-triazole-1-yl)-1-propènes de formule (I) suivant la revendication 1 ou de leurs sels d'addition d'acides et de leurs complexes de sels métalliques compatibles avec les plantes pour combattre des champignons.

6. Utilisation de 3-(1,2,4-triazole-1-yl)-1-propènes de formule (I) suivant la revendication 1 ou de leurs sels d'addition d'acides ou de leurs complexes de sels métalliques compatibles avec les plantes comme agents de protection des plantes.

7. Procédé de préparation de compositions fongicides, caractérisé en ce qu'on mélange des 3-(1,2,4-triazole-1-yl)-1-propènes de formule (I) suivant la revendication 1 ou leurs sels d'addition d'acides ou leurs complexes de sels métalliques compatibles avec les plantes, avec des diluants et/ou des agents tensio-actifs.